# EUROPEAN PATENT APPLICATION

(11) **EP 4 295 837 A1**
(43) Date of publication of application: **27.12.2023**
(21) Application number: 22461576.5
(22) Date of filing: 21.06.2022
(51) Int. Cl.: A61K 9/08, A61K 9/00, A61K 47/14, A61K 47/26, A61K 31/498

(54) **OPHTHALMIC COMPOSITION COMPRISING BRIMONIDINE**

(71) Applicant: Warszawskie Zaklady Farmaceutyczne Polfa S.A., 01-207 Warszawa (PL)
(72) Inventor: POPIELSKA, Iwona, 02-315 Warszawa (PL); RATAJCZAK, Tomasz, 02-315 Warszawa (PL); ROCHOWCZYK, Anna, 02-315 Warszawa (PL)
(74) Representative: Obrycki, Pawel Andrzej

(57) **Abstract**

The present invention relates to preservative-free ophthalmic compositions comprising low-dose of brimonidine.

## Description

### Technical field

The present invention relates to preservative-free ophthalmic compositions comprising low-dose of brimonidine.

### State of the art

Brimonidine is an alpha-adrenergic agonist and 2-imidazoline derivative. Brimonidine is used to treat open-angle glaucoma, ocular hypertension and rosacea. Brimonidine reduces an aqueous humor production and increases uveoscleral outflow. Because it is oxidatively stable, brimonidine is associated with fewer reports of ocular allergic reactions compared to other alpha-2 adrenergic agonists.

Brimonidine is available on the US market under the brand name Lumify as over the counter eye drops comprising brimonidine in a concertation of 0.025 %. It is intended to relieve redness of the eye due to minor eye irritations. Lumify contains brimonidine tartrate, boric acid, sodium borate decahydrate, calcium chloride dihydrate, glycerin, potassium chloride, sodium chloride, benzalkonium chloride as a preservative, purified water, hydrochloric acid or sodium hydroxide for pH adjustment.

Pharmaceutical drugs are most commonly applied to the eye in the form of ophthalmic solutions. There are numbers of quality requirements for the eye drops that need to comply with the physiological parameters of the eye surface. Surface tension is one of the parameters.

Tear film is considered to be a three-layered structure, comprising a mucoidal basal layer, an aqueous component and a superficial lipid layer. Functionally, the three major components of tear film work together to maintain the overall form. Tear film is a nourishing, lubricating and protecting layer that bathes the ocular surface. It is continuously replenished through cycles of production and elimination via evaporation, absorption and drainage. The surface tension of tear film is found to be between 40 mN/m to 46 mN/m which ensures the optimal tear film stability as well as its optimal breakup time on the eyeball.

It is well known that tear film stability is influenced by surface active substances contained in the eye drops. Each ophthalmic ingredient including the preservatives, solubilizing agents and thickening agents can influence the surface tension of the final formulation and can solubilize the tear film lipid layer thereby increasing surface tension of tear fluid and reducing tear film stability. In addition, complex ophthalmic vehicles might cause significant instability of tear film depending on their composition and overall physicochemical properties. ear film stability is of critical importance for the function and comfort of the eye surface. The instability of tear film and shortened time of its breakup can cause the damage of the eye surface and irritative symptoms (e.g. the sensation of sand in the eye, itching, and the sensation of dryness) *(*Hotujac Grgurević M. et al.: Tear fluid - eye drops compatibility assessment using surface tension. Drug development and industrial pharmacy*).* Therefore, surface tension of ophthalmic liquids is an important parameter for quality control processes.

Preservatives are added to eye drops in order to increase shelf life of these medications. A variety of different preservatives may be used in ophthalmic preparations. Examples of preservatives used in ophthalmological compositions are summarized in Table 1. However, these preservatives may have some adverse effects on the eye including irritation of the eye caused by damage of the epithelial cells.

**Table 1. Examples of preservatives used in ophthalmic formulations**

| Compound class | Example |
|---|---|
| Quaternary ammoniums | benzalkonium chloride, polyquaternium-1 |
| Mercurial | thimerosal, phenyl mercuric nitrate, phenyl mercuric acetate |
| Alcohols | chlorbutanol, benzyl alcohol |
| Carboxylic acid | sorbic acid |
| Phenols | methyl/propyl paraben |
| Amidines | chlorhexidine |

The most common preservative used in ocular formulations is benzalkonium chloride (BAC). Benzalkonium chloride possess properties of a cationic surfactant. Benzalkonium chloride has been found to increase the corneal penetration of a number of drugs by emulsification and disruption of cell junctions in the corneal epithelium. BAC works by adhering to the cell membrane of microorganism and increases their permeability which leads to cell lysis. It has been recognized that mammalian cells are unable to neutralize BAC and corneal epithelium is damaged by its entrance. This induces cytotoxic damage to conjunctival cells and stroma. In vitro and in vivo studies demonstrate that BAC can induce toxic and inflammatory effects on the ocular surface. The preservative effect of benzalkonium chloride can be enhanced by boric acid.

Boric acid is used as an antimicrobial preservative and can be used as a buffering agent to control pH. Additionally, it can have the function of tonicity adjusting agent. Borate compounds include boric acid, salts of boric acid and boron oxide and are classified as toxic to reproduction. Therefore, the European Medicine Agency considered that it is necessary to include appropriate warning information in the package leaflet of boron-containing medicinal products, especially with regard to most sensitive populations, i.e. pregnant women and children.

Faced with the problem of providing a composition having an optimal surface tension, the skilled person would turn to use benzalkonium chloride as this compound in addition to its antibacterial properties also acts as a cationic surfactant which reduces surface tension.

Thus, there is still a need to provide a topical composition of brimonidine or pharmaceutically acceptable salt thereof having surface tension that is similar to the surface tension of tear fluid and showing no toxicological effects associated with the presence of preservatives.

### Object of the invention

The object of the present invention is an aqueous ophthalmic composition comprising brimonidine or acceptable salt thereof in an amount of 0.025 % (w/v), characterized in that the composition is preservative free and comprises at least one tonicity agent, at least one buffering agent, and a surfactant selected from the group consisting of macrogolglycerol hydroxystearate, polysorbate 20, polysorbate 80, polyoxyl-40-stearate, propylene glycol, polyethylene glycol (15)-hydroxystearate, 2-[[10,13-dimethyl-17-(6-methylheptan-2-yl)-2,3,4,7,8,9,11,12,14,15,16,17-dodecahydro-1H-cyclopenta[a]phenanthren-3-yl]oxy]ethanol and polyvinyl alcohol.

Surprisingly, it was found that compositions according to the invention have an optimal surface tension which corresponds to surface tension of tear film and provide more homogenous exposition of the drug to the eyeball, therefore enhance the comfort of the users when applying to the eye.

### Description of the invention

Unless otherwise indicated, all ingredients concentrations are listed on a weight/volume percentage basis % (w/v).

Accordingly, the present invention provides an aqueous topical ophthalmic pharmaceutical composition comprising brimonidine or pharmaceutically acceptable salt thereof in an amount of 0.025 % (w/v), at least one tonicity agent, at least one buffering agent and a surfactant that is selected from the group consisting of macrogolglycerol hydroxystearate, polysorbate 20, polysorbate 80, polyoxyl-40-stearate, propylene glycol, polyethylene glycol (15)-hydroxystearate, 2-[[10,13-dimethyl-17-(6-methylheptan-2-yl)-2,3,4,7,8,9,11,12,14,15,16,17-dodecahydro-1H-cyclopenta[a]phenanthren-3-yl]oxy]ethanol and polyvinyl alcohol.

Brimonidine is preferably used in the composition of the present invention in the form of a salt. Preferably, brimonidine is in the form of tartrate salt.

Brimonidine or pharmaceutically acceptable salt thereof is present in the composition according to the present invention in an amount of 0.025 % (w/v).

When used, excipients and carriers incorporated into the composition of the invention must be ophthalmologically acceptable. 'Ophthalmologically acceptable excipients or carriers' refers to ophthalmologically acceptable materials, compositions or vehicles. Each component must be ophthalmologically acceptable in the sense of being compatible with the other ingredients of the ophthalmic composition.

Tonicity agents are added to adjust tonicity of the composition to ophthalmically acceptable levels.

The tonicity agent in the composition according to the invention can be selected from the group consisting of sodium chloride, potassium chloride, magnesium chloride hexahydrate, calcium chloride dihydrate, mannitol and mixtures thereof.

In a preferred embodiment, the composition comprises mannitol and sodium chloride as tonicity agents.

In another embodiment, the composition according to the present invention comprises mannitol, sodium chloride and potassium chloride as tonicity agents.

In yet another embodiment of the present invention, the composition comprises mannitol, sodium chloride, potassium chloride and calcium chloride dihydrate as tonicity agents.

In yet another preferred embodiment, the composition comprises mannitol, sodium chloride, potassium chloride and magnesium chloride hexahydrate as tonicity agents.

In another preferred embodiment, the composition comprises mannitol, sodium chloride, potassium chloride, calcium chloride dihydrate and magnesium chloride hexahydrate as tonicity agents.

In yet another embodiment of the present invention, the composition comprises sodium chloride as tonicity agent.

Surfactant, as used herein, refers to a surface active agent which improves the solubility of a substance, e.g. an active or drug, in a solvent or a substance that acts as a surfactant. For the purpose of the present invention polyvinyl alcohol is considered as a surfactant. Examples of surfactants that may be used in the ophthalmic composition of the present invention include macrogolglycerol hydroxystearate, polysorbate 20, polysorbate 80, polyoxyl - 40 - stearate, propylene glycol, polyethylene glycol (15)-hydroxystearate, 2-[[10,13-dimethyl-17-(6-methylheptan-2-yl)-2,3,4,7,8,9,11,12,14,15,16,17-dodecahydro-1H-cyclopenta[a]phenanthren-3-yl]oxy]ethanol and polyvinyl alcohol.

Macrogolglycerol hydroxystearate also known as Cremophor RH-40 is a non-ionic solubilizer and emulsifying agent obtained by reacting 1 mole of hydrogenated castor oil with 40 moles of ethylene oxide. Macrogolglycerol hydroxystearate is present in the composition according to the present invention in an amount of 0.02 to 0.35 % (w/v).

Polysorbates are a combination of oleic esters of sorbitol and sorbitanes with polyethylene glycol chains. Polysorbate 20 is also known as Tween 20. It is a polyoxyethylene (20) sorbitan monolaureate. Polysorbate 80 is also known as Tween 80. It is a polyoxyethylene (20) sorbitan monooleate. Polysorbate 20 is present in the composition according to the present invention in an amount of 0.01 to 0.25%. Polysorbate 80 is present in the composition according to the present invention in an amount of 0.01 to 0.25 % (w/v)

2-[[10,13-dimethyl-17-(6-methylheptan-2-yl)-2,3,4,7,8,9,11,12,14,15,16,17-dodecahydro-1H-cyclopenta[a]phenanthren-3-yl]oxy]ethanol is also known as Solulan C-24. Solulan C-24 is a lanolin derivative. It is a 100% active complex of ethoxylated cholesterol and ethoxylated vegetable fatty alcohol.

Polyvinyl alcohol is a water-soluble synthetic polymer. It is commonly used to increase viscosity in pharmaceuticals and as a lubricant and protectant in ophthalmic preparations. In the composition according to the present invention polyvinyl alcohol acts as a surfactant and decreases the surface tension.

Ophthalmic composition of the present invention include an effective amount of a buffering agent. Buffers are added to stabilize pH at a level at which drugs are soluble, active, and tolerable. Examples of buffering agents that may be used in the ophthalmic composition of the present invention include citric acid, citrates, phosphoric acid, phosphates, acetates and mixtures thereof. In a preferred embodiment of the present invention, the buffering agent is selected from citric acid, sodium citrate monohydrate, phosphoric acid, sodium or potassium phosphate, dibasic sodium phosphate, sodium or potassium acetate and mixtures thereof. More preferably, the buffering agent is citric acid and sodium citrate monohydrate.

Compositions of the present invention may contain a pH adjusting agent, preferably in an amount sufficient to obtain a composition having a pH of 5.0 - 6.5 Preferably, compositions of the present invention have a pH of 6.4. Strong acids such as hydrochloride acid and strong bases such as sodium hydroxide may be used to adjust pH.

In one embodiment, the composition according to the present invention consists of brimonidine tartrate, sodium citrate, citric acid monohydrate, mannitol, sodium chloride, potassium chloride, macrogolglycerol hydroxystearate, and hydrochloric acid and sodium hydroxide for pH adjustment. The concentration of macrogolglycerol hydroxystearate will range from 0.1 to 0.25 % (w/v).

In another embodiment of the present invention, the composition consist of brimonidine tartrate, sodium citrate, citric acid monohydrate, mannitol, sodium chloride, potassium chloride, polysorbate 80, purified water, and hydrochloric acid and sodium hydroxide for pH adjustment. The concentration of the polysorbate 80 will range from 0.05 to 0.35 % (w/v).

In another embodiment of the present invention, the composition consists of brimonidine tartrate, sodium citrate, citric acid monohydrate, mannitol, sodium chloride, potassium chloride, calcium chloride dihydrate, macrogolglycerol hydroxystearate, purified water, and hydrochloric acid and sodium hydroxide for pH adjustment. The concentration of macrogolglycerol hydroxystearate is 0.25 % (w/v).

In yet another embodiment of the present invention, the composition consists of brimonidine tartrate, sodium citrate, citric acid monohydrate, mannitol, sodium chloride, potassium chloride, magnesium chloride hexahydrate, macrogolglycerol hydroxystearate, purified water, and hydrochloric acid and sodium hydroxide for pH adjustment. The concentration of macrogolglycerol hydroxystearate is 0.25 % (w/v).

In yet another embodiment of the present invention, the composition consists of brimonidine tartrate, sodium citrate, citric acid monohydrate, mannitol, sodium chloride, potassium chloride, magnesium chloride hexahydrate, calcium chloride dihydrate, macrogolglycerol hydroxystearate, purified water, and hydrochloric acid and sodium hydroxide for pH adjustment. The concentration of macrogolglycerol hydroxystearate is 0.25 % (w/v).

In one embodiment, the composition further comprises a polymer of a natural origin as a viscosity agent. Polymers that can be used as viscosity agents in the present inventions are carboxymethyl cellulose sodium (anionic), sodium hyaluronate (anionic), xanthan gum (anionic), chitosan (cationic), guar gum (non-ionic) and sodium alginate (anionic).

Carboxymethyl cellulose sodium/ Carmellose sodium (CMC - Na) is an anionic polymer widely used in oral and topical formulations, primarily for its viscosity increasing properties. Typical molecular weight of sodium carboxymethyl cellulose is 90,000-700,000 Da. Most preferred for use in the composition of the present invention is Aqualon CMC 7L2P PH BET. The concentration of CMC-Na in the composition of the present invention will range from 0.6 to 0.7 % (w/v), and will preferably be 0.7 % (w/v).

In one embodiment, the composition consists of brimonidine tartrate, sodium citrate, citric acid monohydrate, mannitol, sodium chloride, macrogolglycerol hydroxystearate, and hydrochloric acid and sodium hydroxide for pH adjustment. The concentration of macrogolglycerol hydroxystearate will range from 0.15 to 0.25 % (w/v).

In yet another embodiment, the composition consist of brimonidine tartrate, sodium citrate, citric acid monohydrate, mannitol, sodium chloride, polysorbate 80, and hydrochloric acid and sodium hydroxide for pH adjustment. The concentration of polysorbate 80 will range from 0.2 to 0.4 % (w/v).

Guar gum is a galactomannan, commonly used in cosmetics, food products, and pharmaceutical formulations. Guar gum consists of linear chains of (1→4)-β-D-mannopyranosyl units with α-D-galactopyranosyl units attached by (1→6) linkages. The ratio of D-galactose to D-mannose is between 1:1.4 and 1:2. The Ph.Eur describes guar galactomannan as being obtained from the seeds of Cyamopsis tetragonolobus (L.) Taub. by grinding the endosperms and subsequent partial hydrolysis.

The concentration of guar gum in the composition of the present invention will range from 0.05 to 0.1 %, and will preferably be 0.1 %. In one embodiment, the composition consists of brimonidine tartrate, guar gum, sodium citrate, citric acid monohydrate, sodium chloride, macrogolglycerol hydroxystearate, hydrochloric acid, sodium hydroxide and purified water. The concentration of macrogolglycerol hydroxystearate will range from 0.25 to 0.35 % (w/v).

Xanthan gum is a high molecular weight polysaccharide gum. It contains D-glucose and D-mannose as the dominant hexose units, along with D-glucuronic acid, and is prepared as the sodium, potassium, or calcium salt. Xanthan gum is widely used in oral and topical pharmaceutical formulations, cosmetics, and foods as a suspending and stabilizing agent. It is also used as a thickening and emulsifying agent. It is nontoxic, compatible with most other pharmaceutical ingredients, and has good stability and viscosity properties over a wide pH and temperature range. Xanthan gum is a polysaccharide produced by a pure-culture aerobic fermentation of a carbohydrate with Xanthomonas campestris. The polysaccharide is then purified by recovery with propan-2-ol, dried, and milled.

The concentration of xanthan gum in the composition of the present invention will range from 0.03 to 0.04 % (w/v). In one embodiment of the present invention, the composition consists of brimonidine tartrate, sodium citrate, citric acid monohydrate, sodium chloride, xanthan gum, macrogolglycerol hydroxystearate, hydrochloric acid, sodium hydroxide and purified water. The concentration of macrogolglycerol hydroxystearate in the composition of the invention will range from 0.25 to 0.35 % (w/v).

Hyaluronic acid is a natural substance which can be found in the eye but also in other parts of the body. It is a long-chain polymer of disaccharide units of glucuronic acid and N-acetylglucosamine linked via alternating β-(1→4) and β-(1→3) glycosidic bonds. Hyaluronic acid can be 25,000 disaccharide repeats in length. Polymers of hyaluronic acid can range in size from 5,000 to 20,000,000 Da in vivo.

Several ophthalmologically acceptable salts of hyaluronic acid are known. The most common is sodium hyaluronate. Most preferred for use in the composition of the present invention is sodium hyaluronate obtained from Contipro company having a molecular weight of 0.34 MDa.

The concentration of sodium hyaluronate in the composition of the invention will range from 0.15 to 0.2 % (w/v). In one embodiment of the present invention, the composition consists of brimonidine tartrate, mannitol, sodium citrate, citric acid monohydrate, sodium hyaluronate, macrogolglycerol hydroxystearate, hydrochloric acid, sodium hydroxide and purified water. The concentration of macrogolglycerol hydroxystearate in the composition of the invention will range from 0.2 to 0.25 % (w/v). Such compositions ensure that a uniform, stable and particularly long-term-adhering moisture film is formed on the surface on the eye which cannot be rinsed off rapidly.

Chitosan is a linear polysaccharide composed of randomly distributed β-(1→4)-linked D-glucosamine (deacetylated unit) and N-acetyl-D-glucosamine (acetylated unit). It is made by treating the chitin shells of shrimp and other crustaceans with an alkaline substance, such as sodium hydroxide. The concentration of chitosan in the composition of the present invention will range from 0.1 to 0.15 % (w/v).

In one embodiment of the present invention, the composition consists of brimonidine tartrate, chitosan, sodium citrate, citric acid monohydrate, sodium chloride, macrogoglycerol hydroxystearate, hydrochloric acid, sodium hydroxide and purified water. The concentration of macrogolglycerol hydroxystearate in the composition of the invention will range from 0.15 to 0.25 % (w/v).

Sodium alginate is obtained mainly from algae belonging to the Phaeophyceae. It consists mainly of the sodium salt of alginic acid which is a mixture of pluronic acids [(C₆H₈O₆)ₙ] composed of units of D-mannuronic acid and L-guluronic acid.

The concentration of sodium alginate in the composition of the present invention will range from 0.2 to 0.3 % (w/v). In yet another embodiment, the composition consists of brimonidine tartrate, sodium citrate, citric acid monohydrate, sodium alginate, sodium chloride, macrogolglycerol hydroxystearate, hydrochloric acid, sodium hydroxide and purified water. The concentration of macrogolglycerol hydroxystearate in the composition of the invention will range from 0.15 to 0.25 % (w/v).

According to a particularly preferred embodiment, the composition according to the present invention is devoid of preservatives. By preservative it is understood any substance which can be used as a ophthalmological preservative, e.g. benzalkonium chloride, polyquaternium-1, thimerosal, phenyl mercuric nitrate, phenyl mercuric acetate, chlorbutanol, benzyl alcohol, sorbic acid, methyl/propyl paraben, disodium EDTA. It has been recognized that such preservatives may cause many side effects including irritation of the eye caused by damage of the epithelial cells.

The ophthalmic composition of the present invention can be packed in suitable single dose containers or a multi-dose containers. In a preferred embodiment the composition of the present invention is packed in a low density polyethylene container (LDPE), advantageously of EP quality containing no additives. Non-limiting examples of multi-dose container to deliver eye drops without preservatives for several days are Novelia^{®}, or 3K^{®} system.

The surface tension of the pharmaceutical composition according to present invention is preferably 42 mN/m to 46 mN/m. The surface tension is a value measured by methods known to those skilled in the art, for example by the EasyDyne KRUSS K20 device using the Wilhelmy plate method with a small volume measuring vessel.

The following examples are provided to illustrate different aspects and embodiments of the present invention. These examples are not intended to limit in any way the disclosed invention.

### Example 1

The ophthalmic composition was prepared using the following steps:
a) The container was filled with 0.8 ml of purified water having a temperature of 25°C and 7 mg of carboxymethyl cellulose sodium was added and mixed. Dissolution was continued for at least 1 hour.
b) 5.36 mg of sodium citrate, 0.42 mg of citric acid monohydrate and 7 mg of sodium chloride were added to the solution obtained in step a) and mixed. Dissolution was continued for at least 40 minutes.
c) 0.25 mg of brimonidine tartrate was added to the solution obtained in step b). Dissolution was continued for 30 minutes.
d) Another container was filled with highly purified water having a temperature of 25°C and 2.5 mg of macrogolglycerol hydroxystearate was added and mixed. Dissolution was continued for 20 minutes.
e) The solution obtained in step d) was transferred to the solution obtained in step c).
f) The pH of the solution obtained in step e) was adjusted by using sodium hydroxide solution (10%) or hydrochloric acid, dilute (10%) to 6.4 and highly purified water was added to bring the final volume up to 1 ml.
g) The solution obtained in step f) was filtered through 0.2 µm membrane directly to the sterile empty container;

| **Ingredient** | **Each 1 ml of solution contains** |
|---|---|
| Brimonidine tartrate | 0.25 mg |
| Carboxymethyl cellulose sodium | 7.00 mg |
| Sodium citrate | 5.36 mg |
| Citric acid monohydrate | 0.42 mg |
| Sodium chloride | 7.00 mg |
| Macrogolglycerol hydroxystearate | 2.50 mg |
| Hydrochloric acid, dilute | q.s. pH 5.7-6.5 |
| Sodium hydroxide | q.s. pH 5.7-6.5 |
| Purified water | ad 1 ml |

### Example 2

The ophthalmic composition was prepared using the following steps:
a) The container was filled with 0.8 ml of purified water having a temperature of 25°C and 7 mg of carboxymethyl cellulose sodium was added and mixed. Dissolution was continued for at least 1 hour.
b) 5.36 mg of sodium citrate, 0.42 mg of citric acid monohydrate and 7 mg of sodium chloride were added to the solution obtained in step a) and mixed. Dissolution was continued for at least 40 minutes.
c) 0.25 mg of brimonidine tartrate was added to the solution obtained in step b). Dissolution was continued for 30 minutes.
d) Another container was filled with highly purified water having a temperature of 25°C and 2 mg of macrogolglycerol hydroxystearate was added and mixed. Dissolution was continued for 20 minutes.
e) The solution obtained in step d) was transferred to the solution obtained in step c).
f) The pH of the solution obtained in step e) was adjusted by using sodium hydroxide solution (10%) or hydrochloric acid, dilute (10%) to 6.4 and highly purified water was added to bring the final volume up to 1 ml.
g) The solution obtained in step f) was filtered through 0.2 µm membrane directly to the sterile empty container;

| **Ingredient** | **Each 1 ml of solution contains** |
|---|---|
| Brimonidine tartrate | 0.25 mg |
| carboxymethyl cellulose sodium | 7.00 mg |
| Sodium citrate | 5.36 mg |
| Citric acid monohydrate | 0.42 mg |
| Sodium chloride | 7.00 mg |
| Macrogolglycerol hyd roxystearate | 2 mg |
| Hydrochloric acid, dilute | q.s. pH 5.7-6.5 |
| Sodium hydroxide | q.s. pH 5.7-6.5 |
| Purified water | ad 1 ml |

### Example 3

The ophthalmic composition was prepared using the following steps:
a) The container was filled with 0.8 ml purified water having a temperature of 25°C and 7 mg of carboxymethyl cellulose sodium was added and mixed. Dissolution was continued for at least 1 hour.
b) 5.36 mg of sodium citrate, 0.42 mg of citric acid monohydrate and 7 mg of sodium chloride were added to the solution obtained in step a) and mixed. Dissolution was continued for at least 40 minutes.
c) 0.25 mg of brimonidine tartrate was added to the solution obtained in step b). Dissolution was continued for 30 minutes.
d) Another container was filled with highly purified water having a temperature of 25°C and 1.5 mg of macrogolglycerol hydroxystearate was added and mixed. Dissolution was continued for 20 minutes.
e) The solution obtained in step d) was transferred to the solution obtained in step c).
f) The pH of the solution was adjusted by using sodium hydroxide solution (10%) or hydrochloric acid, dilute (10%) to 6.4 and highly purified water was added to bring the final volume up to 1 ml.
g) The solution obtained in step f) was filtered through 0.2 µm membrane directly to the sterile empty container;

| **Ingredient** | **Each 1 ml of solution contains** |
|---|---|
| Brimonidine tartrate | 0.25 mg |
| Carboxymethyl cellulose sodium | 7.00 mg |
| Sodium citrate | 5.36 mg |
| Citric acid monohydrate | 0.42 mg |
| Sodium chloride | 7.00 mg |
| Macrogolglycerol hydroxystearate | 1.50 mg |
| Hydrochloric acid, dilute | q.s. pH 5.7-6.5 |
| Sodium hydroxide | q.s. pH 5.7-6.5 |
| Purified water | ad 1 ml |

### Example 4

The ophthalmic composition was prepared using the following steps:
a) The container was filled with 0.8 ml of purified water having a temperature of 25°C and 2 mg of sodium hyaluronate was added and mixed. Dissolution was continued of for at least 1 hour at the temperature of 50°C.
b) 5.36 mg of sodium citrate, 0.42 mg of citric acid monohydrate and 7 mg of sodium chloride were added to the solution obtained in step a) and mixed. Dissolution was continued for at least 1 hour.
c) 0.25 mg of brimonidine tartrate was added to the solution obtained in step b). Dissolution was continued for 30 minutes.
d) Another container was filled with highly purified water having a temperature of 25°C and 2 mg of macrogolglycerol hydroxystearate was added and mixed. Dissolution was continued for 20 minutes.
e) The solution obtained in step d) was transferred to the solution obtained in step c) and mixed for 20 minutes.
f) The pH of the solution obtained in step e) was adjusted by using sodium hydroxide solution (10%) or hydrochloric acid, dilute (10%) to range 5.7 -6.5 and highly purified water was added to bring the final volume up to 1 ml.
g) The solution obtained in step f) was filtered through 0.2 µm membrane directly to the sterile empty container.

| **Ingredient** | **Each 1 ml of solution contains** |
|---|---|
| Brimonidine tartrate | 0.25 mg |
| Sodium hyaluronate | 2.00 mg |
| Sodium citrate | 5.36 mg |
| Citric acid monohydrate | 0.42 mg |
| Sodium chloride | 7.00 mg |
| Macrogolglycerol hydroxystearate | 2 mg |
| Hydrochloric acid, dilute | q.s. pH 5.7-6.5 |
| Sodium hydroxide | q.s. pH 5.7-6.5 |
| Purified water | ad 1 ml |

### Example 5

The ophthalmic composition was prepared using the following steps:
a) The container was filled with 0.8 ml of purified water having a temperature of 25°C and 2 mg of sodium hyaluronate was added and mixed. Dissolution was continued of for at least 1 hour at the temperature of 50°C.
b) 5.36 mg of sodium citrate, 0.42 mg of citric acid monohydrate and 7 mg of sodium chloride were added to the solution obtained in step a) and mixed. Dissolution was continued for at least 1 hour.
c) 0.25 mg of brimonidine tartrate was added to the solution obtained in step b). Dissolution was continued for 30 minutes.
d) Another container was filled with highly purified water having a temperature of 25°C and 2.5 mg of macrogolglycerol hydroxystearate was added and mixed. Dissolution was continued for 20 minutes.
e) The solution obtained in step d) was transferred to the solution obtained in step c) and mixed for 20 minutes.
f) The pH of the solution was adjusted by using sodium hydroxide solution (10%) or hydrochloric acid, dilute (10%) to range 6.4 and highly purified water was added to bring the final volume up to 1 ml.
g) The solution obtained in step f) was filtered through 0.2 µm membrane directly to the sterile empty container.

| **Ingredient** | **Each 1 ml of solution contains** |
|---|---|
| Brimonidine tartrate | 0.25 mg |
| Sodium hyaluronate | 2.00 mg |
| Sodium citrate | 5.36 mg |
| Citric acid monohydrate | 0.42 mg |
| Sodium chloride | 7.00 mg |
| Macrogolglycerol hydroxystearate | 2.50 mg |
| Hydrochloric acid, dilute | q.s. pH 5.7-6.5 |
| Sodium hydroxide | q.s. pH 5.7-6.5 |
| Purified water | ad 1 ml |

### Example 6

The ophthalmic composition was prepared using the following steps:
a) The container was filled with 0.8 ml of purified water having a temperature of 25°C and 0.4 mg of xanthan gum was added and mixed. Dissolution was continued of for at least 1 hour.
b) 5.36 mg of sodium citrate, 0.42 mg of citric acid monohydrate and 7 mg of sodium chloride were added to the solution obtained in step a) and mixed. Dissolution was continued for at least 1 hour.
c) 0.25 mg of brimonidine tartrate was added to the solution obtained in step b). Dissolution was continued for 30 minutes.
d) Another container was filled with highly purified water having a temperature of 25°C and 2.5 mg of macrogolglycerol hydroxystearate was added and mixed. Dissolution was continued for 20 minutes.
e) The solution obtained in step d) was transferred to the solution obtained in step c) and mixed for 20 minutes.
f) The pH of the solution obtained in step e) was adjusted by using sodium hydroxide solution (10%) or hydrochloric acid, dilute (10%) to range 6.4 and highly purified water was added to bring the final volume up to 1 ml.
g) The solution obtained in step f) was filtered through 0.2 µm membrane directly to the sterile empty container.

| **Ingredient** | **Each 1 ml of solution contains** |
|---|---|
| Brimonidine tartrate | 0.25 mg |
| Xanthan gum | 0.40 mg |
| Sodium citrate | 5.36 mg |
| Citric acid monohydrate | 0.42 mg |
| Sodium chloride | 7.00 mg |
| Macrogolglycerol hydroxystearate | 2.50 mg |
| Hydrochloric acid, dilute | q.s. pH 5.7-6.5 |
| Sodium hydroxide | q.s. pH 5.7-6.5 |
| Purified water | ad 1 ml |

### Example 7

The ophthalmic composition was prepared using the following steps:
a) The container was filled with 0.8 ml of purified water having a temperature of 25°C and 0.4 mg of xanthan gum was added and mixed. Dissolution was continued of for at least 1 hour.
b) 5.36 mg of sodium citrate, 0.42 mg of citric acid monohydrate and 7 mg of sodium chloride were added to the solution obtained in step a) and mixed. Dissolution was continued for at least 1 hour.
c) 0.25 mg of brimonidine tartrate was added to the solution obtained in step b). Dissolution was continued for 30 minutes.
d) Another container was filled with highly purified water having a temperature of 25°C and 3 mg of macrogolglycerol hydroxystearate was added and mixed. Dissolution was continued for 20 minutes.
e) The solution obtained in step d) was transferred to the solution obtained in step c) and mixed for 20 minutes.
f) The pH of the solution obtained in step e) was adjusted by using sodium hydroxide solution (10%) or hydrochloric acid, dilute (10%) to range 6.4 and highly purified water was added to bring the final volume up to 1 ml.
g) The solution obtained in step f) was filtered through 0.2 µm membrane directly to the sterile empty container.

| Ingredient | Each 1 ml of solution contains |
|---|---|
| Brimonidine tartrate | 0.25 mg |
| Xanthan gum | 0.40 mg |
| Sodium citrate | 5.36 mg |
| Citric acid monohydrate | 0.42 mg |
| Sodium chloride | 7.00 mg |
| Macrogolglycerol hydroxystearate | 3 mg |
| Hydrochloric acid, dilute | q.s. pH 5.7-6.5 |
| Sodium hydroxide | q.s. pH 5.7-6.5 |
| Purified | ad 1 ml |

### Example 8

The ophthalmic composition was prepared using the following steps:
a) The container was filled with 0.8 ml of purified water having a temperature of 25°C and 0.4 mg of xanthan gum was added and mixed. Dissolution was continued of for at least 1 hour.
b) 5.36 mg of sodium citrate, 0.42 mg of citric acid monohydrate and 7 mg of sodium chloride were added to the solution obtained in step a) and mixed. Dissolution was continued for at least 1 hour.
c) 0.25 mg of brimonidine tartrate was added to the solution obtained in step b). Dissolution was continued for 30 minutes.
d) Another container was filled with highly purified water having a temperature of 25°C and 3.5 mg of macrogolglycerol hydroxystearate was added and mixed. Dissolution was continued for 20 minutes.
e) The solution obtained in step d) was transferred to the solution obtained in step c) and mixed for 20 minutes.
f) The pH of the solution obtained in step e) was adjusted by using sodium hydroxide solution (10%) or hydrochloric acid, dilute (10%) to 6.4 and highly purified water was added to bring the final volume up to 1 ml.
g) The solution obtained in step f) was filtered through 0.2 µm membrane directly to the sterile empty container.

| Ingredient | Each 1 ml of solution contains |
|---|---|
| Brimonidine tartrate | 0.25 mg |
| Xanthan gum | 0.40 mg |
| Sodium citrate | 5.36 mg |
| Citric acid monohydrate | 0.42 mg |
| Sodium chloride | 7.00 mg |
| Macrogolglycerol hydroxystearate | 3.50 mg |
| Hydrochloric acid, dilute | q.s. pH 5.7-6.5 |
| Sodium hydroxide | q.s. pH 5.7-6.5 |
| Purified water | ad 1 ml |

### Example 9

The ophthalmic composition was prepared using the following steps:
a) The container was filled with 0.8 ml of purified water having a temperature of 25°C, then guar gum was added while continuously mixing. Dissolution was continued at least 1 hour at the temp. 60°C. After dispersing of guar gum the solution was cooled and homogenized Then sodium citrate, citric acid monohydrate, sodium chloride were added and dissolved while continuously mixing. Dissolution was continued for at least 30 minutes. Then brimonidine tartrate was added, dissolution was continued for 30 minutes;
b) Another container was filled with highly purified water at the temp. 25°C, then macrogolglycerol hydroxystearate were dissolved while continuously mixing. Dissolution was continued for 20 minutes;
c) The solution as obtained in step (b) was transfer to container with solution (a) while continuously mixing. Mixing was continued for 20 minutes;
d) Optionally adjusting of pH of the solution as obtained in step (c) with a pH adjusting agent sodium hydroxide solution (10%) or hydrochloric acid, dilute (10%). The pH of the solution can then be adjusted to about 6.4 range 4
e) Optionally final adjustment of volume of the solution was performed as obtained in step (d) with highly purified water at the temp. 25°C;
f) Optionally filtering of the solution as obtained in step (e), preferably through 0.2 µm membrane directly to the sterile empty container;
g) Optionally the aseptic filling of the solution from step (f) to sterile containers.

| Ingredient | Each 1 ml of solution contains |
|---|---|
| Brimonidine tartrate | 0.25 mg |
| Guar gum | 1.0 |
| Sodium citrate | 5.36 mg |
| Citric acid monohydrate | 0.42 mg |
| Sodium chloride | 7.00 mg |
| Macrogolglycerol hydroxystearate | 2.50 |
| Hydrochloric acid, dilute | q.s. pH 5.7-6.5 |
| Sodium hydroxide | q.s. pH 5.7-6.5 |
| Purified | ad 1 ml |

### Example 10

The ophthalmic composition was prepared using the following steps:
a) The container was filled in with 0.8 ml of purified water having a temperature of 25 °C. Then 5.36 mg of sodium citrate, 0.42 mg of citric acid monohydrate, 5.9 mg of sodium chloride, 0.3 mg of potassium chloride and 10 mg of mannitol were dissolved in highly purified water and mixed. Dissolution was continued for at least 30 minutes.
b) 0.25 mg of brimonidine tartrate was added to the solution obtained in step a) and dissolution was continued for 30 minutes.
c) Another container was filled in with highly purified water at the temperature of 25°C and 0.5 mg of macrogolglycerol hydroxystearate was dissolved while mixing. Dissolution was continued for 20 minutes.
d) The solution obtained in step c) was added to the solution b) with highly purified water and macrogolglycerol hydroxystearate and mixed for 20 minutes.
e) The pH of the obtained solution was adjusted to 6.4 The volume of the final solution was adjusted to 1 ml by highly purified water.
f) The solution was filtered through 0.2 µm membrane directly to the sterile empty container.

| Ingredient | Each 1 ml of solution contains |
|---|---|
| Brimonidine tartrate | 0.25 mg |
| Mannitol | 10.0 |
| Sodium citrate | 5.36 mg |
| Citric acid monohydrate | 0.42 mg |
| Sodium chloride | 7.00 mg |
| Macrogolglycerol hydroxystearate | 0.50 |
| Hydrochloric acid, dilute | q.s. pH 5.7-6.5 |
| Sodium hydroxide | q.s. pH 5.7-6.5 |
| Purified water | ad 1 ml |

### Example 11

The ophthalmic composition was prepared using the following steps:
a) The container was filled in with 0.8 ml of purified water having a temperature of 25 °C. Then 5.36 mg of sodium citrate, 0.42 mg of citric acid monohydrate, 5.9 mg of sodium chloride, 0.3 mg of potassium chloride and 10 mg of mannitol were dissolved in highly purified water and mixed. Dissolution was continued for at least 30 minutes.
b) 0.25 mg of brimonidine tartrate was added to the solution obtained in step a) and dissolution was continued for 30 minutes.
c) Another container was filled in with highly purified water at the temperature of 25°C and 1 mg of macrogolglycerol hydroxystearate was dissolved while mixing. Dissolution was continued for 20 minutes.
d) The solution obtained in step b) was added to the solution with highly purified water and macrogolglycerol hydroxystearate and mixed for 20 minutes.
e) The pH of the obtained solution was adjusted to 6.4 by adding sodium hydroxide solution (10%) or hydrochloric acid, dilute (10%) . The volume of the final solution was adjusted to 1 ml by highly purified water.
f) The solution was filtered through 0.2 µm membrane directly to the sterile empty container.

| **Ingredient** | **Each 1 ml of solution contains** |
|---|---|
| Brimonidine tartrate | 0.25 mg |
| Mannitol | 10.0 mg |
| Sodium citrate | 5.36 mg |
| Citric acid monohydrate | 0.42 mg |
| Sodium chloride | 5.90 mg |
| Potassium chloride | 0.30 mg |
| Macrogolglycerol hydroxystearate | 1.00 mg |
| Hydrochloric acid, dilute | q.s. pH 5.7-6.5 |
| Sodium hydroxide | q.s. pH 5.7-6.5 |
| Purified water | ad 1 ml |

### Example 12

The ophthalmic composition was prepared using the following steps:
a) The container was filled in with 0.8 ml of purified water at having a temperature of 25 °C. Then 5.36 mg of sodium citrate, 0.42 mg of citric acid monohydrate, 5.9 mg of sodium chloride, 0.3 mg of potassium chloride and 10 mg of mannitol were dissolved in highly purified water and mixed. Dissolution was continued for at least 30 minutes.
b) 0.25 mg of brimonidine tartrate was added to the solution obtained in step a) and dissolution was continued for 30 minutes.
c) Another container was filled in with highly purified water at the temperature of 25°C and 1.5 mg of macrogolglycerol hydroxystearate was dissolved while mixing. Dissolution was continued for 20 minutes.
d) The solution obtained in step b) was added to the solution with highly purified water and macrogolglycerol hydroxystearate and mixed for 20 minutes.
e) The pH of the obtained solution was adjusted to 6.4 by adding sodium hydroxide solution (10%) or hydrochloric acid, dilute (10%) . The volume of the final solution was adjusted to 1 ml by highly purified water.
f) The solution was filtered through 0.2 µm membrane directly to the sterile empty container.

| **Ingredient** | **Each 1 ml of solution contains** |
|---|---|
| Brimonidine tartrate | 0.25 mg |
| Mannitol | 10.0 mg |
| Sodium citrate | 5.36 mg |
| Citric acid monohydrate | 0.42 mg |
| Sodium chloride | 5.90 mg |
| Potassium chloride | 0.30 mg |
| Macrogolglycerol hydroxystearate | 1.50 mg |
| Hydrochloric acid, dilute | q.s. pH 5.7-6.5 |
| Sodium hydroxide | q.s. pH 5.7-6.5 |
| Purified water | ad 1 ml |

### Example 13

The ophthalmic composition was prepared using the following steps:
a) The container was filled in with 0.8 ml of purified water having a temperature of 25 °C. Then 5.36 mg of sodium citrate, 0.42 mg of citric acid monohydrate, 5.9 mg of sodium chloride, 0.3 mg of potassium chloride and 10 mg of mannitol were dissolved in highly purified water and mixed. Dissolution was continued for at least 30 minutes.
b) 0.25 mg of brimonidine tartrate was added to the solution obtained in step a) and dissolution was continued for 30 minutes.
c) Another container was filled in with highly purified water at the temperature of 25°C and 2 mg of macrogolglycerol hydroxystearate was dissolved while mixing. Dissolution was continued for 20 minutes.
d) The solution obtained in step b) was added to the solution with highly purified water and macrogolglycerol hydroxystearate and mixed for 20 minutes.
e) The pH of the obtained solution was adjusted to 6.4 by adding sodium hydroxide solution (10%) or hydrochloric acid, dilute (10%) . The volume of the final solution was adjusted to 1 ml by highly purified water.
f) The solution was filtered through 0.2 µm membrane directly to the sterile empty container.

| **Ingredient** | **Each 1 ml of solution contains** |
|---|---|
| Brimonidine tartrate | 0.25 mg |
| Mannitol | 10.0 mg |
| Sodium citrate | 5.36 mg |
| Citric acid monohydrate | 0.42 mg |
| Sodium chloride | 5.90 mg |
| Potassium chloride | 0.30 mg |
| Macrogolglycerol hydroxystearate | 2.00 mg |
| Hydrochloric acid, dilute | q.s. pH 5.7-6.5 |
| Sodium hydroxide | q.s. pH 5.7-6.5 |
| Purified water | ad 1 ml |

### Example 14

The ophthalmic composition was prepared using the following steps:
a) The container was filled in with 0.8 ml of purified water having a temperature of 25 °C. Then 5.36 mg of sodium citrate, 0.42 mg of citric acid monohydrate, 5.9 mg of sodium chloride, 0.3 mg of potassium chloride and 10 mg of mannitol were dissolved in highly purified water and mixed. Dissolution was continued for at least 30 minutes.
b) 0.25 mg of brimonidine tartrate was added to the solution obtained in step a) and dissolution was continued for 30 minutes.
c) Another container was filled in with highly purified water at the temperature of 25°C and 2.5 mg of macrogolglycerol hydroxystearate was dissolved while mixing. Dissolution was continued for 20 minutes.
d) The solution obtained in step b) was added to the solution with highly purified water and macrogolglycerol hydroxystearate and mixed for 20 minutes.
e) The pH of the obtained solution was adjusted to 6.4 by adding sodium hydroxide solution (10%) or hydrochloric acid, dilute (10%). The volume of the final solution was adjusted to 1 ml by highly purified water.
f) The solution was filtered through 0.2 µm membrane directly to the sterile empty container.

| **Ingredient** | **Each 1 ml of solution contains** |
|---|---|
| Brimonidine tartrate | 0.25 mg |
| Mannitol | 10.0 mg |
| Sodium citrate | 5.36 mg |
| Citric acid monohydrate | 0.42 mg |
| Sodium chloride | 5.90 mg |
| Potassium chloride | 0.30 mg |
| Macrogolglycerol hydroxystearate | 2.50 mg |
| Hydrochloric acid, dilute | q.s. pH 5.7-6.5 |
| Sodium hydroxide | q.s. pH 5.7-6.5 |
| Purified water | ad 1 ml |

### Example 15

The ophthalmic composition was prepared using the following steps:
a) The container was filled in with 0.8 ml of purified water having a temperature of 25 °C. Then 5.36 mg of sodium citrate, 0.42 mg of citric acid monohydrate, 5.9 mg of sodium chloride, 0.3 mg of potassium chloride, 0.19 mg of calcium chloride dihydrate and 10 mg of mannitol were dissolved in highly purified water and mixed. Dissolution was continued for at least 30 minutes.
b) 0.25 mg of brimonidine tartrate was added to the solution obtained in step a) and dissolution was continued for 30 minutes.
c) Another container was filled in with highly purified water at the temperature of 25°C and 2.5 mg of macrogolglycerol hydroxystearate was dissolved while mixing. Dissolution was continued for 20 minutes.
d) The solution obtained in step b) was added to the solution with highly purified water and macrogolglycerol hydroxystearate and mixed for 20 minutes.
e) The pH of the obtained solution was adjusted to 6.4 by adding sodium hydroxide solution (10%) or hydrochloric acid, dilute (10%). The volume of the final solution was adjusted to 1 ml by highly purified water.
f) The solution was filtered through 0.2 µm membrane directly to the sterile empty container.

| **Ingredient** | **Each 1 ml of solution contains** |
|---|---|
| Brimonidine tartrate | 0.25 mg |
| Mannitol | 10.0 mg |
| Sodium citrate | 5.36 mg |
| Citric acid monohydrate | 0.42 mg |
| Sodium chloride | 5.30 mg |
| Potassium chloride | 0.30 mg |
| Calcium chloride dihydrate | 0.19 mg |
| Macrogolglycerol hydroxystearate | 2.50 mg |
| Hydrochloric acid, dilute | q.s. pH 5.7-6.5 |
| Sodium hydroxide | q.s. pH 5.7-6.5 |
| Purified water | ad 1 ml |

### Example 16

The ophthalmic composition was prepared using the following steps:
a) The container was filled in with 0.8 ml of purified water having a temperature of 25 °C. Then 5.36 mg of sodium citrate, 0.42 mg of citric acid monohydrate, 5.9 mg of sodium chloride, 0.3 mg of potassium chloride, 0.19 mg of magnesium chloride hexahydrate and 10 mg of mannitol were dissolved in highly purified water and mixed. Dissolution was continued for at least 30 minutes.
b) 0.25 mg of brimonidine tartrate was added to the solution obtained in step a) and dissolution was continued for 30 minutes.
c) Another container was filled in with highly purified water at the temperature of 25°C and 2.5 mg of macrogolglycerol hydroxystearate was dissolved while mixing. Dissolution was continued for 20 minutes.
d) The solution obtained in step b) was added to the solution with highly purified water and macrogolglycerol hydroxystearate and mixed for 20 minutes.
e) The pH of the obtained solution was adjusted to 6.4 by adding sodium hydroxide solution (10%) or hydrochloric acid, dilute (10%). The volume of the final solution was adjusted to 1 ml by highly purified water.
f) The solution was filtered through 0.2 µm membrane directly to the sterile empty container.

| **Ingredient** | **Each 1 ml of solution contains** |
|---|---|
| Brimonidine tartrate | 0.25 mg |
| Mannitol | 10.0 mg |
| Sodium citrate | 5.36 mg |
| Citric acid monohydrate | 0.42 mg |
| Sodium chloride | 5.30 mg |
| Potassium chloride | 0.30 mg |
| Magnesium chloride hexahydrate | 0.19 mg |
| Macrogolglycerol hydroxystearate | 2.50 mg |
| Hydrochloric acid, dilute | q.s. pH 5.7-6.5 |
| Sodium hydroxide | q.s. pH 5.7-6.5 |
| Purified water, | ad 1 ml |

### Example 17

The ophthalmic composition was prepared using the following steps:
a) The container was filled in with 0.8 ml of purified water having a temperature of 25 °C. Then 5.36 mg of sodium citrate, 0.42 mg of citric acid monohydrate, 5.9 mg of sodium chloride, 0.3 mg of potassium chloride, 0.19 mg of calcium chloride dihydrate, 0.19 mg of magnesium chloride hexahydrate and 10 mg of mannitol were dissolved in highly purified water and mixed. Dissolution was continued for at least 30 minutes.
b) 0.25 mg of brimonidine tartrate was added to the solution obtained in step a) and dissolution was continued for 30 minutes.
c) Another container was filled in with highly purified water at the temperature of 25°C and 2.5 mg of macrogolglycerol hydroxystearate was dissolved while mixing. Dissolution was continued for 20 minutes.
d) The solution obtained in step b) was added to the solution with highly purified water and macrogolglycerol hydroxystearate and mixed for 20 minutes.
e) The pH of the obtained solution was adjusted to 6.4 by adding sodium hydroxide solution (10%) or hydrochloric acid, dilute (10%). The volume of the final solution was adjusted to 1 ml by highly purified water.
f) The solution was filtered through 0.2 µm membrane directly to the sterile empty container.

| **Ingredient** | **Each 1 ml of solution contains** |
|---|---|
| Brimonidine tartrate | 0.25 mg |
| Mannitol | 10.0 mg |
| Sodium citrate | 5.36 mg |
| Citric acid monohydrate | 0.42 mg |
| Sodium chloride | 5.30 mg |
| Potassium chloride | 0.30 mg |
| Calcium chloride dihydrate | 0.19 mg |
| Magnesium chloride hexahydrate | 0.19 mg |
| Macrogolglycerol hydroxystearate | 2.50 mg |
| Hydrochloric acid, dilute | q.s. pH 5.7-6.5 |
| Sodium hydroxide | q.s. pH 5.7-6.5 |
| Purified water | ad 1 ml |

### Example 18

The ophthalmic composition was prepared using the following steps:
a) The container was filled in with 0.8 ml of purified water having a temperature of 25 °C. Then 5.36 mg of sodium citrate, 0.42 mg of citric acid monohydrate, 5.9 mg of sodium chloride, 0.3 mg of potassium chloride and 10 mg of mannitol were dissolved in highly purified water and mixed. Dissolution was continued for at least 30 minutes.
b) 0.25 mg of brimonidine tartrate was added to the solution obtained in step a) and dissolution was continued for 30 minutes.
c) Another container was filled in with highly purified water at the temperature of 25°C and 0.5 mg of polysorbate 80 was dissolved while mixing. Dissolution was continued for 20 minutes.
d) The solution obtained in step b) was added to the solution with highly purified water and polysorbate 80 and mixed for 20 minutes.
e) The pH of the obtained solution was adjusted to 6.4 by adding sodium hydroxide solution (10%) or hydrochloric acid, dilute (10%).The volume of the final solution was adjusted to 1 ml by highly purified water.
f) The solution was filtered through 0.2 µm membrane directly to the sterile empty container.

| **Ingredient** | **Each 1 ml of solution contains** |
|---|---|
| Brimonidine tartrate | 0.25 mg |
| Mannitol | 10.0 mg |
| Sodium citrate | 5.36 mg |
| Citric acid monohydrate | 0.42 mg |
| Sodium chloride | 5.90 mg |
| Potassium chloride | 0.30 mg |
| Polysorbate 80 | 0.50 mg |
| Hydrochloric acid, dilute | q.s. pH 5.7-6.5 |
| Sodium hydroxide | q.s. pH 5.7-6.5 |
| Purified water | ad 1 ml |

### Example 19

The ophthalmic composition was prepared using the following steps:
a) The container was filled in with 0.8 ml of purified water having a temperature of 25 °C. Then 5.36 mg of sodium citrate, 0.42 mg of citric acid monohydrate, 5.9 mg of sodium chloride, 0.3 mg of potassium chloride and 10 mg of mannitol were dissolved in highly purified water and mixed. Dissolution was continued for at least 30 minutes.
b) 0.25 mg of brimonidine tartrate was added to the solution obtained in step a) and dissolution was continued for 30 minutes.
c) Another container was filled in with highly purified water at the temperature of 25°C and 1 mg of polysorbate 80 was dissolved while mixing. Dissolution was continued for 20 minutes.
d) The solution obtained in step b) was added to the solution with highly purified water and polysorbate 80 and mixed for 20 minutes.
e) The pH of the obtained solution was adjusted to 6.4 by adding sodium hydroxide solution (10%) or hydrochloric acid, dilute (10%).. The volume of the final solution was adjusted to 1 ml by highly purified water.
f) The solution was filtered through 0.2 µm membrane directly to the sterile empty container.

| **Ingredient** | **Each 1 ml of solution contains** |
|---|---|
| Brimonidine tartrate | 0.25 mg |
| Mannitol | 10.0 mg |
| Sodium citrate | 5.36 mg |
| Citric acid monohydrate | 0.42 mg |
| Sodium chloride | 5.90 mg |
| Potassium chloride | 0.30 mg |
| Polysorbate 80 | 1.00 mg |
| Hydrochloric acid, dilute | q.s. pH 5.7-6.5 |
| Sodium hydroxide | q.s. pH 5.7-6.5 |
| Purified water | ad 1 ml |

### Example 20

The ophthalmic composition was prepared using the following steps:
a) The container was filled in with 0.8 ml of purified water having a temperature of 25 °C. Then 5.36 mg of sodium citrate, 0.42 mg of citric acid monohydrate, 5.9 mg of sodium chloride, 0.3 mg of potassium chloride and 10 mg of mannitol were dissolved in highly purified water and mixed. Dissolution was continued for at least 30 minutes.
b) 0.25 mg of brimonidine tartrate was added to the solution obtained in step a) and dissolution was continued for 30 minutes.
c) Another container was filled in with highly purified water at the temperature of 25°C and 1.5 mg of polysorbate 80 was dissolved while mixing. Dissolution was continued for 20 minutes.
d) The solution obtained in step b) was added to the solution with highly purified water and polysorbate 80 and mixed for 20 minutes.
e) The pH of the obtained solution was adjusted to 6.4 by adding sodium hydroxide solution (10%) or hydrochloric acid, dilute (10%).. The volume of the final solution was adjusted to 1 ml by highly purified water.
f) The solution was filtered through 0.2 µm membrane directly to the sterile empty container.

| **Ingredient** | **Each 1 ml of solution contains** |
|---|---|
| Brimonidine tartrate | 0.25 mg |
| Mannitol | 10.0 mg |
| Sodium citrate | 5.36 mg |
| Citric acid monohydrate | 0.42 mg |
| Sodium chloride | 5.90 mg |
| Potassium chloride | 0.30 mg |
| Polysorbate 80 | 1.50 mg |
| Hydrochloric acid, dilute | q.s. pH 5.7-6.5 |
| Sodium hydroxide | q.s. pH 5.7-6.5 |
| Purified water | ad 1 ml |

### Example 21

The ophthalmic composition was prepared using the following steps:
a) The container was filled in with purified water having a temperature of 25 °C. Then 5.36 mg of sodium citrate, 0.42 mg of citric acid monohydrate, 5.9 mg of sodium chloride, 0.3 mg of potassium chloride and 10 mg of mannitol were dissolved in highly purified water and mixed. Dissolution was continued for at least 30 minutes.
b) 0.25 mg of brimonidine tartrate was added to the solution obtained in step a) and dissolution was continued for 30 minutes.
c) Another container was filled in with highly purified water at the temperature of 25°C and 2 mg of polysorbate 80 was dissolved while mixing. Dissolution was continued for 20 minutes.
d) The solution obtained in step b) was added to the solution with highly purified water and polysorbate 80 and mixed for 20 minutes.
e) The pH of the obtained solution was adjusted to 6.4 by adding sodium hydroxide solution (10%) or hydrochloric acid, dilute (10%).. The volume of the final solution was adjusted to 1 ml by highly purified water.
f) The solution was filtered through 0.2 µm membrane directly to the sterile empty container.

| **Ingredient** | **Each 1 ml of solution contains** |
|---|---|
| Brimonidine tartrate | 0.25 mg |
| Mannitol | 10.0 mg |
| Sodium citrate | 5.36 mg |
| Citric acid monohydrate | 0.42 mg |
| Sodium chloride | 5.90 mg |
| Potassium chloride | 0.30 mg |
| Polysorbate 80 | 2.00 mg |
| Hydrochloric acid, dilute | q.s. pH 5.7-6.5 |
| Sodium hydroxide | q.s. pH 5.7-6.5 |
| Purified water | ad 1 ml |

### Example 22

The ophthalmic composition was prepared using the following steps:
a) The container was filled in with highly purified water at the temperature of 25 °C. Then 5.36 mg of sodium citrate, 0.42 mg of citric acid monohydrate, 5.9 mg of sodium chloride, 0.3 mg of potassium chloride and 10 mg of mannitol were dissolved in highly purified water and mixed. Dissolution was continued for at least 30 minutes.
b) 0.25 mg of brimonidine tartrate was added to the solution obtained in step a) and dissolution was continued for 30 minutes.
c) Another container was filled in with highly purified water at the temperature of 25°C and 2.5 mg of polysorbate 80 was dissolved while mixing. Dissolution was continued for 20 minutes.
d) The solution obtained in step b) was added to the solution with highly purified water and polysorbate 80 and mixed for 20 minutes.
e) The pH of the obtained solution was adjusted to 6.4 by adding sodium hydroxide solution (10%) or hydrochloric acid, dilute (10%).. The volume of the final solution was adjusted to 1 ml by highly purified water.
f) The solution was filtered through 0.2 µm membrane directly to the sterile empty container.

| **Ingredient** | **Each 1 ml of solution contains** |
|---|---|
| Brimonidine tartrate | 0.25 mg |
| Mannitol | 10.0 mg |
| Sodium citrate | 5.36 mg |
| Citric acid monohydrate | 0.42 mg |
| Sodium chloride | 5.90 mg |
| Potassium chloride | 0.30 mg |
| Polysorbate 80 | 2.5 mg |
| Hydrochloric acid, dilute | q.s. pH 5.7-6.5 |
| Sodium hydroxide | q.s. pH 5.7-6.5 |
| Purified water | ad 1 ml |

### Example 23

The ophthalmic composition was prepared using the following steps:
a) The container was filled in with 0.8 ml of purified water having a temperature of 25 °C. Then 5.36 mg of sodium citrate, 0.42 mg of citric acid monohydrate, 5.9 mg of sodium chloride, 0.3 mg of potassium chloride and 10 mg of mannitol were dissolved in highly purified water and mixed. Dissolution was continued for at least 30 minutes.
b) 0.25 mg of brimonidine tartrate was added to the solution obtained in step a) and dissolution was continued for 30 minutes.
c) Another container was filled in with highly purified water at the temperature of 25°C and 3 mg of polysorbate 80 was dissolved while mixing. Dissolution was continued for 20 minutes.
d) The solution obtained in step b) was added to the solution with highly purified water and polysorbate 80 and mixed for 20 minutes.
e) The pH of the obtained solution was adjusted to 6.4 by adding sodium hydroxide or hydrochloric acid. The volume of the final solution was adjusted to 1 ml by highly purified water.
f) The solution was filtered through 0.2 µm membrane directly to the sterile empty container.

| **Ingredient** | **Each 1 ml of solution contains** |
|---|---|
| Brimonidine tartrate | 0.25 mg |
| Mannitol | 10.0 mg |
| Sodium citrate | 5.36 mg |
| Citric acid monohydrate | 0.42 mg |
| Sodium chloride | 5.90 mg |
| Potassium chloride | 0.30 mg |
| Polysorbate 80 | 3.00 mg |
| Hydrochloric acid, dilute | q.s. pH 5.7-6.5 |
| Sodium hydroxide | q.s. pH 5.7-6.5 |
| Purified water | ad 1 ml |

### Example 24

The ophthalmic composition was prepared using the following steps:
a) The container was filled in with 0.8 ml of purified water having a temperature of 25 °C. Then 5.36 mg of sodium citrate, 0.42 mg of citric acid monohydrate, 5.9 mg of sodium chloride, 0.3 mg of potassium chloride and 10 mg of mannitol were dissolved in highly purified water and mixed. Dissolution was continued for at least 30 minutes.
b) 0.25 mg of brimonidine tartrate was added to the solution obtained in step a) and dissolution was continued for 30 minutes.
c) Another container was filled in with highly purified water at the temperature of 25°C and 3.5 mg of polysorbate 80 was dissolved while mixing. Dissolution was continued for 20 minutes.
d) The solution obtained in step b) was added to the solution with highly purified water and polysorbate 80 and mixed for 20 minutes.
e) The pH of the obtained solution was adjusted to 6.4 by adding sodium hydroxide solution (10%) or hydrochloric acid, dilute (10%).. The volume of the final solution was adjusted to 1 ml by highly purified water.
f) The solution was filtered through 0.2 µm membrane directly to the sterile empty container.

| **Ingredient** | **Each 1 ml of solution contains** |
|---|---|
| Brimonidine tartrate | 0.25 mg |
| Mannitol | 10.0 mg |
| Sodium citrate | 5.36 mg |
| Citric acid monohydrate | 0.42 mg |
| Sodium chloride | 5.90 mg |
| Potassium chloride | 0.30 mg |
| Polysorbate 80 | 3.50 mg |
| Hydrochloric acid, dilute | q.s. pH 5.7-6.5 |
| Sodium hydroxide | q.s. pH 5.7-6.5 |
| Purified water | ad 1 ml |

### Example 25

The ophthalmic composition was prepared using the following steps:
a) The container was filled with 0.8 ml of purified water having a temperature of 25°C. Then 5.36 mg of sodium citrate, 0.42 mg of citric acid monohydrate, 7.0 mg of sodium chloride were added in highly purified water and mixed. Dissolution was continued for at least 30 minutes.
b) 14.0 mg poly(vinyl) alcohol was added while continuously mixing. Dissolution was continued for 2-3 hours at the temp. 75°C. After dissolution of PVA the solution was cooled.
c) 0.25 mg of brimonidine tartrate was added to the solution obtained in step b). Dissolution was continued for 30 minutes.
d) The pH of the solution obtained in step c) was adjusted by using sodium hydroxide solution (10%) or hydrochloric acid, dilute (10%) to range 5.7 -6.5 and highly purified water was added to bring the final volume up to 1 ml.
e) The solution obtained in step d) was filtered through 0.2 µm membrane directly to the sterile empty container.

| **Ingredient** | **Each 1 ml of solution contains** |
|---|---|
| Brimonidine tartrate | 0.25 mg |
| Poly(vinyl alcohol) 40-88 | 14.00 mg |
| Sodium citrate | 5.36 mg |
| Citric acid monohydrate | 0.42 mg |
| Sodium chloride | 7.00 mg |
| Hydrochloric acid, dilute | q.s. pH 5.7-6.5 |
| Sodium hydroxide | q.s. pH 5.7-6.5 |
| Purified water | ad 1 ml |

### Example 26

The ophthalmic composition was prepared using the following steps:
a) The container was filled with 0.8 ml of purified water at having a temperature of 25°C, then 7 mg of carboxymethyl cellulose sodium was added while continuously mixing. Dissolution was continued for at least 1 hour. Then 5.36 mg of sodium citrate, 0.42 mg of citric acid monohydrate, 7 mg of sodium chloride were added and dissolved while continuously mixing. Dissolution was continued for 40 minutes; T
b) 0.25 mg of brimonidine tartrate was added to the solution obtained in step a) and dissolution was continued for 30 minutes;
c) Another container was filled with highly purified water at the temp. 25°C, then 0.2 mg of polisorbate 80 was dissolved while continuously mixing. Dissolution was continued for 20 minutes;
d) The solution as obtained in step (b) was transfered to container with solution (a) while continuously mixing. Mixing was continued for 20 minutes;
e) The pH of the solution obtained in step c) was adjusted to 6.4 Optionally adjusting of pH of the solution as obtained in step (c) with a pH adjusting agent. by adding sodium hydroxide solution (10%) or hydrochloric acid, dilute (10%).. The volume of the final solution was adjusted to 1 ml by highly purified water;
f) The solution was filtered through 0.2 µm membrane directly to the sterile empty container.

| **Ingredient** | **Each 1 ml of solution contains** |
|---|---|
| Brimonidine tartrate | 0.25 mg |
| Carboxymethyl cellulose sodium | 7.00 mg |
| Sodium citrate | 5.36 mg |
| Citric acid monohydrate | 0.42 mg |
| Sodium chloride | 7.00 mg |
| Polysorbate 80 | 0.20 mg |
| Hydrochloric acid, dilute | q.s. pH 5.7-6.5 |
| Sodium hydroxide | q.s. pH 5.7-6.5 |
| Purified water | ad 1 ml |

### Example 27

The ophthalmic composition was prepared using the following steps:
a) The container was filled with 0.8 ml of purified water having a temperature of 25°C, then 7 mg of carboxymethyl cellulose sodium was added while continuously mixing. Dissolution was continued for at least 1 hour. Then 5.36 mg of sodium citrate, 0.42 mg of citric acid monohydrate, 7 mg of sodium chloride were added and dissolved while continuously mixing. Dissolution was continued for 40 minutes; T
b) 0.25 mg of brimonidine tartrate was added to the solution obtained in step a) and dissolution was continued for 30 minutes;
c) Another container was filled with highly purified water at the temp. 25°C, then 0.1 mg of polysorbate 80 was dissolved while continuously mixing. Dissolution was continued for 20 minutes;
d) The solution as obtained in step (b) was transferred to container with solution (a) while continuously mixing. Mixing was continued for 20 minutes;
e) The pH of the solution obtained in step c) was adjusted to 6.4 Optionally adjusting of pH of the solution as obtained in step (c) with a pH adjusting agent. by adding sodium hydroxide solution (10%) or hydrochloric acid, dilute (10%). The volume of the final solution was adjusted to 1 ml by highly purified water;
f) The solution was filtered through 0.2 µm membrane directly to the sterile empty container.

| **Ingredient** | **Each 1 ml of solution contains** |
|---|---|
| Brimonidine tartrate | 0.25 mg |
| Carboxymethyl cellulose sodium | 7.00 mg |
| Sodium citrate | 5.36 mg |
| Citric acid monohydrate | 0.42 mg |
| Sodium chloride | 7.00 mg |
| Polysorbate 80 | 0.3 mg |
| Hydrochloric acid, dilute | q.s. pH 5.7-6.5 |
| Sodium hydroxide | q.s. pH 5.7-6.5 |
| Purified water | ad 1 ml |

### Example 28

The ophthalmic composition was prepared using the following steps:
a) The container was filled with 0.8 ml of purified water having a temperature of 25°C, then 7 mg of carboxymethyl cellulose sodium was added while continuously mixing. Dissolution was continued for at least 1 hour. Then 5.36 mg of sodium citrate, 0.42 mg of citric acid monohydrate, 7 mg of sodium chloride were added and dissolved while continuously mixing. Dissolution was continued for 40 minutes; T
b) 0.25 mg of brimonidine tartrate was added to the solution obtained in step a) and dissolution was continued for 30 minutes;
c) Another container was filled with highly purified water at the temp. 25°C, then 0.4 mg of polysorbate 80 was dissolved while continuously mixing. Dissolution was continued for 20 minutes;
d) The solution as obtained in step (b) was transferred to container with solution (a) while continuously mixing. Mixing was continued for 20 minutes;
e) The pH of the solution obtained in step c) was adjusted to 6.4 Optionally adjusting of pH of the solution as obtained in step (c) with a pH adjusting agent. by adding sodium hydroxide or hydrochloric acid. The volume of the final solution was adjusted to 1 ml by highly purified water;
f) The solution was filtered through 0.2 µm membrane directly to the sterile empty container.

| **Ingredient** | **Each 1 ml of solution contains** |
|---|---|
| Brimonidine tartrate | 0.25 mg |
| Carboxymethyl cellulose sodium | 7.00 mg |
| Sodium citrate | 5.36 mg |
| Citric acid monohydrate | 0.42 mg |
| Sodium chloride | 7.00 mg |
| Polysorbate 80 | 0.4 |
| Hydrochloric acid, dilute | q.s. pH 5.7-6.5 |
| Sodium hydroxide | q.s. pH 5.7-6.5 |
| Purified water | ad 1 ml |

### Example 29

The ophthalmic composition was prepared using the following steps:
a) The container was filled with 0.7 ml of purified water having a temperature of 25°C, then 1.5 mg of chitosan was added while continuously mixing. Dispersing of chitosan was continued for 30 minutes;
b) Hydrochloric acid was added to the solution obtained in step a)
c) 5.36 mg of sodium citrate, 0.42 mg of citric acid monohydrate, 7 mg of sodium chloride were added and dissolved while continuously mixing. Dissolution was continued for 30 minutes;
d) The pH of the solution obtained in step c) was adjusted to 6.4;
e) 0.25 mg of brimonidine tartrate was added and dissolution was continued for 30 minutes;
f) Another container was filled with highly purified water at the temp. 25°C, then 0.025 mg of macrogolglycerol hydroxystearate was dissolved while continuously mixing. Dissolution was continued for 20 minutes;
g) The solution obtained in step (f) was transferred to container with solution (e) while continuously mixing. Mixing was continued for 20 minutes;
h) The pH of the solution obtained in step c) was adjusted to 6.4 by adding sodium hydroxide or hydrochloric acid. The volume of the final solution was adjusted to 1 ml by highly purified water;
i) The solution was filtered through 0.2 µm membrane directly to the sterile empty container.

| **Ingredient** | **Each 1 ml of solution contains** |
|---|---|
| Brimonidine tartrate | 0.25 mg |
| Chitosan | 1.5 mg |
| Sodium citrate | 5.36 mg |
| Citric acid monohydrate | 0.42 mg |
| Sodium chloride | 7.00 mg |
| Macrogolglycerol hydroxystearate | 2.5 mg |
| Hydrochloric acid, dilute | q.s. pH 5.7-6.5 |
| Sodium hydroxide | q.s. pH 5.7-6.5 |
| Purified water | ad 1 ml |

### Example 30

The ophthalmic composition was prepared using the following steps:
a) The container was filled with 0.8 ml of purified water having a temperature of 25°C, then 3 mg of sodium alginate was added while continuously mixing. Dissolution was continued for at least 1 hour. Then 5.36 mg of sodium citrate, 0.42 mg of citric acid monohydrate, 7 mg of sodium chloride were added and dissolved while continuously mixing. Dissolution was continued for 40 minutes; T
b) 0.25 mg of brimonidine tartrate was added to the solution obtained in step a) and dissolution was continued for 30 minutes;
c) Another container was filled with highly purified water at the temp. 25°C, then 0.5 mg of macrogolglycerol hydroxystearate was dissolved while continuously mixing. Dissolution was continued for 20 minutes;
d) The solution as obtained in step (c) was transferred to container with solution (b) while continuously mixing. Mixing was continued for 20 minutes;
e) The pH of the solution was adjusted to 6.4. The volume of the final solution was adjusted to 1 ml by highly purified water;
f) The solution was filtered through 0.2 µm membrane directly to the sterile empty container.

| **Ingredient** | **Each 1 ml of solution contains** |
|---|---|
| Brimonidine tartrate | 0.25 mg |
| Sodium alginate* | 3.0 mg |
| Sodium citrate | 5.36 mg |
| Citric acid monohydrate | 0.42 mg |
| Sodium chloride | 7.0 mg |
| Macrogolglycerol hydroxystearate | 1.5 |
| Hydrochloric acid, dilute | q.s. pH 5.7-6.5 |
| Sodium hydroxide | q.s. pH 5.7-6.5 |
| Purified water | ad 1 ml |

### Example 31

The ophthalmic composition was prepared using the following steps:
a) The container was filled with 0.8 ml of purified water having a 25°C, then 3 mg of sodium alginate was added while continuously mixing. Dissolution was continued for at least 1 hour. Then 5.36 mg of sodium citrate, 0.42 mg of citric acid monohydrate, 7 mg of sodium chloride were added and dissolved while continuously mixing. Dissolution was continued for 40 minutes; T
b) 0.25 mg of brimonidine tartrate was added to the solution obtained in step a) and dissolution was continued for 30 minutes;
c) Another container was filled with highly purified water at the temp. 25°C, then 0.5 mg of macrogolglycerol hydroxystearate was dissolved while continuously mixing. Dissolution was continued for 20 minutes;
d) The solution as obtained in step (c) was transferred to container with solution (b) while continuously mixing. Mixing was continued for 20 minutes;
e) The pH of the solution was adjusted to 6.4. The volume of the final solution was adjusted to 1 ml by highly purified water;
f) The solution was filtered through 0.2 µm membrane directly to the sterile empty container.

| **Ingredient** | **Each 1 ml of solution contains** |
|---|---|
| Brimonidine tartrate | 0.25 mg |
| Sodium alginate* | 3.0 mg |
| Sodium citrate | 5.36 mg |
| Citric acid monohydrate | 0.42 mg |
| Sodium chloride | 7.0 mg |
| Macrogolglycerol hydroxystearate | 2.5 mg |
| Hydrochloric acid, dilute | q.s. pH 5.7-6.5 |
| Sodium hydroxide | q.s. pH 5.7-6.5 |
| Purified water | ad 1 ml |

### Results

### Degree of coloration of liquids.

Incompatibility studies were performed in order to determine interactions of the drug substance with other components of formulations. Compositions from examples 1 to 28 were prepared and degree of coloration of liquids was examined by visual method according to the standard of PH. Eur. 2.2.1. The colors were compared in diffused daylight viewing horizontally (method I) and vertically (method II) against a white background.

No substantial changes in physical appearance of the products were observed. All tested composition were transparent which proves compatibility of ingredients.

### Surface tension

As comparative examples 1-3 composition of LUMIFY^{®} (brimonidine tartrate, boric acid, sodium borate decahydrate, calcium chloride dihydrate, glycerin, potassium chloride, sodium chloride, benzalkonium chloride as a preservative, purified water, hydrochloric acid or sodium hydroxide) from 3 commercially available batches were tested.

The surface tension of was tested with the EasyDyne KRUSS K20 device using the Wilhelmy plate method with a small volume measuring vessel. The measurement was done at 22±2°C. The correctness of measurements of the tensiometer operation was correlated with the tests of surface tension of high purified water as a reference sample. Compositions from examples 1-28 as well as compositions from comparative examples 1-3 were tested.

**Table 2. Surface tension of tested compositions**

| **Composition** | **Surface tension mN/m** |
|---|---|
| Composition 1 | 46.3 |
| Composition 2 | 46.8 |
| Composition 3 | 46.9 |
| Composition 4 | 47.4 - |
| Composition 5 | 46.6 |
| Composition 6 | 46.9 |
| Composition 7 | 46.7 |
| Composition 8 | 46.9 |
| Composition 9 | 47.9 |
| Composition 10 | 49.0 |
| Composition 11 | 46.7 |
| Composition 12 | 46.5 |
| Composition 13 | 46.3 |
| Composition 14 | 45.8 |
| Composition 15 | 46.6 |
| Composition 16 | 46.7 |
| Composition 17 | 46.4 |
| Composition 18 | 46.8 |
| Composition 19 | 45.9 |
| Composition 20 | 44.2 |
| Composition 21 | 43.8 |
| Composition 22 | 43.6 |
| Composition 23 | 43.2 |
| Composition 24 | 43.1 |
| Composition 25 | 51.4? |
| Composition 26 | 47.1 |
| Composition 27 | 46.3 |
| Composition 28 | 46.0 |
| Composition 29 | 46.1 |
| Composition 30 | 49.3 |
| Composition 31 | 46.5 |
| Comparative example 1 | 43.5 |
| Comparative example 2 | 45 |
| Comparative example 3 | 45.2 |

This data suggests that all tested composition have surface tension that is very close to the surface tension of tear film which is found to be between 42 mN/m to 46 mN/m. Values below the lower end point and above the higher end point may also be acceptable. This ensures the optimal tear film stability as well as its optimal breakup time on the eyeball.

Due to the presence of benzalkonium chloride the compositions of Lumify (comparative examples 1-3) have surface tension that is similar to the surface tension of a tear film. However, the presence of benzalkonium chloride has an adverse effect on the eyeball and causes damages of epithelial cells.

It is to be noted that compositions according to present invention are much favorable as they show similar surface tension to the preserved composition and at the same time do not demonstrate toxic and inflammatory effects on the epithelial cells.

## Claims

1. An aqueous ophthalmic composition comprising brimonidine or acceptable salt thereof in an amount of 0.025 % (w/v), **characterized in that** the composition is preservative-free and comprises at least one tonicity agent, at least one buffering agent, and a surfactant selected from the group consisting of macrogolglycerol hydroxystearate, polysorbate 20, polysorbate 80, polyoxyl-40-stearate, propylene glycol, polyethylene glycol (15)-hydroxystearate, 2-[[10,13-dimethyl-17-(6-methylheptan-2-yl)-2,3,4,7,8, 9,11,12,14,15,16,17-dodecahydro-1H-cyclopenta[a]phenanthren-3-yl]oxy]ethanol and polyvinyl alcohol.

2. The composition according to claim 1, wherein the tonicity agent is selected from the group consisting of sodium chloride, potassium chloride, magnesium chloride, calcium chloride, mannitol and mixtures thereof.

3. The composition according to claim 1, wherein the buffering agent is selected from the group consisting of citric acid, sodium citrate, citric acid monohydrate, phosphoric acid, sodium or potassium phosphate, dibasic sodium phosphate, sodium or potassium acetate and mixtures thereof.

4. The composition according to any of the preceding claims, wherein the composition further comprises pH adjusting agents.

5. The composition according to any of the preceding claims, wherein the composition has a pH of 5 to 6.5, preferably 6.4.

6. The composition according to any of the preceding claims, wherein the composition further comprises a viscosity agent selected from the group consisting of carboxymethyl cellulose sodium, sodium hyaluronate, xanthan gum, guar gum, chitosan and sodium alginate.

7. The composition according to claim 6, wherein the viscosity agent is carboxymethyl cellulose sodium used in an amount of 0.6 to 0.7 % (w/v) or sodium hyaluronate used in an amount of 0.15 to 0.2 % (w/v) or sodium alginate used in an amount of 0.2 to 0.3 % (w/v).

8. The composition according to claim 6 or 7, wherein the composition consists of
- Brimonidine tartrate
- Mannitol
- Sodium citrate
- Citric acid monohydrate
- Carboxymethyl cellulose sodium
- Sodium chloride
- Macrogolglycerol hydroxystearate
- Hydrochloric acid
- Sodium hydroxide
- Purified water

9. The composition according to claim 8, wherein the macrogolglycerol hydroxystearate is used in an amount of 0.15 to 0.25 % (w/v).

10. The composition according to claim 6 or 7, wherein the composition consists of
- Brimonidine tartrate
- Mannitol
- Sodium citrate
- Citric acid monohydrate
- Carboxymethyl cellulose sodium
- Sodium chloride
- Polysorbate 80
- Hydrochloric acid
- Sodium hydroxide
- Purified water

11. The composition according to claim 10, wherein the polysorbate 80 is present in an amount of 0.05 to 0.35 % (w/v).

12. The composition according to claim 6 or 7, wherein the composition consists of
- Brimonidine tartrate
- Mannitol
- Sodium citrate
- Citric acid monohydrate
- Sodium hyaluronate
- Sodium chloride
- Macrogolglycerol hydroxystearate
- Hydrochloric acid
- Sodium hydroxide
- Purified water

13. The composition according to claim 12, wherein the macrogolglycerol hydroxystearate is present in an amount of 0.2 to 0.25 % (w/v).

14. The composition according to claim 6 or 7, wherein the composition consists of
- Brimonidine tartrate
- Sodium alginate
- Sodium citrate
- Citric acid monohydrate
- Sodium chloride
- Macrogolglycerol hydroxystearate
- Hydrochloric acid
- Sodium hydroxide
- Purified water

15. The composition according to claim 14, wherein the macrogolglycerol hydroxystearate is present in an amount of 0.05 to 0.25 % (w/v).
